# EUROPEAN PATENT SPECIFICATION

(11) **EP 3 258 894 B1**
(45) Date of publication and mention of the grant of the patent: **12.08.2020**
(21) Application number: 16706155.5
(22) Date of filing: 15.02.2016
(51) Int. Cl.: A61F 9/04

(54) **EYE PATCH AND METHOD FOR DETECTING MOVEMENT DATA AND/OR POSITION DATA USING THE EYE PATCH**
AUGENKLAPPE UND VERFAHREN ZUR ERMITTLUNG VON BEWEGUNGS- UND/ODER LAGEDATEN UNTER VERWENDUNG DER AUGENKLAPPE
CACHE OEIL ET PROCÉDÉ DE DÉTECTION DE MOUVEMENT ET / OU DE DONNÉES SPATIALES PAR USAGE DUDIT CACHE OEIL

(30) Priority: 19.02.2015 DE 102015102373
(43) Date of publication of application: 27.12.2017
(73) Proprietor: Prahs, Philipp, 93049 Regensburg (DE)
(72) Inventor: Prahs, Philipp, 93049 Regensburg (DE)
(74) Representative: Benninger, Johannes
(86) International application number: PCT/EP2016/053170
(87) International publication number: WO 2016/131774

(56) References cited:
- DE-A1-102010 046 291
- FR-A1- 2 896 986
- US-A- 4 850 376
- US-A- 5 879 292
- US-B1- 8 494 507

## Description

The present invention relates to an eye patch with a position sensor and/or with a movement sensor having the features of the independent claim 1 as well as to a method according to the independent method claim 9 for the continuous detection of movement data and/or of position data using the eye patch. Further aspects and preferred embodiments are defined in the appended claims. Aspects, embodiments and examples of the present disclosure which do not fall under the scope of the appended claims do not form part of the invention and are merely provided for illustrative purposes.

In ophthalmology, there is a multitude of situations in which it is indicated to position the head so that it is in a stable, unchangeable or as little as possible changeable position. The employment of a so-called intraocular gas tamponade can serve as an example in this context, which gas tamponade is used after surgical procedures at the eyeball, for example in the case of a retinal detachment, where the substance in the eyeball, the vitreous body, is at least partly replaced by a filling of air or gas.

Another example is lamellar keratoplasties, in which a filling of air remains in the anterior chamber of the eye and causes the adhesion of the corneal transplant to the recipient cornea.

On the one hand, such a gas tamponade can support the attachment of the retina to the interior wall of an eyeball, and on the other hand, it can serve to prevent fluids from entering through small holes or the like in the retina by disabling the fluid flow through retinal holes or similar injuries, which fluid flow is caused by capillary effects etc. by way of the surface tension present at the gas-water interface. No further measures are necessary for removing the gas tamponade itself; the gas contained therein, mostly sulfur hexafluoride, decomposes by itself in the course of the healing process by way of resorptive processes, with the diameter of the gas bubble, which was injected into the eyeball, steadily decreasing.

It is therefore understandable that, during the course of healing, in particular, in the instance of a decreasing diameter of the gas bubble, the gas tamponade should be secured as unchangeably as possible in the place intended for the purpose, for example the surgical site. The optimal position of the eye, and thus of the head, even of the entire body, if need be, can certainly be specified by and ophthalmologist. For the wearer of the eye patch, however, it is impossible or difficult to return to the optimal state after a change in position that can occur unintentionally, for instance while sleeping, so that an auxiliary means to enable the wearer to return to that optimal state would be very useful.

US 5 879 292 A and its family member DE 698 16 318 T2 describe a wound dressing in the form of an eye patch, consisting of a compress, a microcontroller, and a sensor. The purpose of the described sensor arrangement lies in monitoring the wear time of the wound dressing, where the sensor, which is integrated in the compress, reports the existing or missing skin contact, respectively, to the microcontroller, which stores the data. These data can then be read out by way of a data interface.

DE 10 2010 038 028 A1 discloses a method for detecting emotional states of a horse, in which method a movement sensor or the like detects the position of the ears, for example, stores this information in an electronic unit and provides the stored data via an interface for evaluation.

WO 2010/038156 A1 relates to a wireless ECG monitoring system for ambulatory patient monitoring. The data are registered by an appropriate sensor technology, digitalized, and passed on to a smartphone for evaluation by means of a suitable software. The software is furthermore suited for transferring status information to remote locations.

WO 2011/035262 A1 shows a method for applying an implantable MEMS sensor in ophthalmology, in which the sensor is designed for measuring the intraocular pressure.

US 8 494 507 B1 relates to a mobile terminal for individuals with disabilities, which terminal comprises a plurality of different sensors and a software interface and can be programmed to evaluate the sensor data and provide an assistive output with regard to the respective disability, based on the programmed software rules.

In view of the procedures known from the prior art, a primary object of the invention lies in providing a device and a method with which the position of the eye or the head, respectively, can be detected as precisely as possible, which enable the ophthalmologist to specify an optimal desired position, and which enable the wearer of the eye patch, by means of optical, acoustic or another type of feedback, to resume the mentioned desired position after a change in position.

It is furthermore the object of the invention to create the conditions for assessing healing successes or the chances therefor on the basis of statistical data by providing the possibility of recording and evaluating the movement data and/or position data.

Besides the possibility of data acquisition, it is finally the object of the eye patch according to the invention to also provide a protective function for the eye covered by the eye patch.

The stated tasks are respectively solved by the objects of the independent claims. Further advantageous embodiments of the invention are described in the subclaims. For fulfilling the stated objects, the present invention proposes an eye patch for at least partially covering a human or animal eye and shielding it from mechanical and/or climatic influences and/or from electromagnetic radiation, in particular from light radiation.

As a rule, the term eye in this context is intended to refer to a human eye, which is undergoing, for instance, a curative treatment or a therapy, or which is convalescing. It is however not excluded to use the eye patch according to the invention or to apply it, respectively, as well as the method according to the invention, for animals, too, since it is generally conceivable to also carry out a curative treatment of an eye for an animal, and to collect the same data as is the case for a human eye.

Furthermore, if covering the eye at least partially is referred to, this should be understood to the effect that the position detection and the use of the eye patch can also function with the eye itself not being completely covered or shielded from the mentioned climatic, mechanical and/or electromagnetic influences. Even if the eye patch normally covers the eye largely or completely, respectively, it is not ruled out that the eye can be only partially covered in individual instances. Be it emphasized that such an instance diminishes neither the possible application of the eye patch according to the invention nor the effectiveness of the method according to the invention.

The eye patch according to the invention has a shell that covers the eye and its surrounding, which shell is made of bending-resistant material, and has a sensor unit or a sensor module, as well, which comprises at least:
- one for the continuous acquisition of movement data of the eye patch by measuring spatial acceleration movements and rotation movements,
- one microcontroller for processing the output signals of the at least one sensor,
- one data interface designed for wireless bidirectional communication, as well as
- one electrical energy storage for the electrical energy supply of at least the microcontroller and the data interface.

Of course, there can also be two or more sensor units or sensor modules, respectively, the data from which are processed by the microcontroller. In addition, the sensor unit or the sensor units, respectively, can also be assigned a data storage unit that is permanently and/or temporarily couplable with the position sensor and/or with the movement sensor for the purpose of the storage of the movement data and/or of the position data detected by the sensor or sensors, respectively. The sensor device or the sensor unit, respectively (also: the plurality of sensor devices or sensor units, respectively), can be arranged in or structurally integrated into the eye patch according to the invention, respectively; the sensor unit, which is adapted to the common contour of such eye patches, serves for the detection of the movement data and/or of the position data and/or for the storage of the detected data or for the transmission of the detected data, respectively, which can in turn support the desired healing process in the above mentioned eye surgeries. The eye patch that is equipped in such a manner is used instead of the eye patch commonly applied for this purpose and is thus able to not only fulfill the function assigned to it of covering the eye as a protection from mechanical adverse effects, such as contact, contamination, etc., but also of supporting the healing success by sensor support and by support from acoustic or other electronic auxiliary means. According to the embodiment, i.e. to the size and form of the sensor device, it can also be possible to additionally provide a standard eye patch with the sensor device, which in this instance can be fastened, optionally also by means of detachable fastening devices, to the outside of the eye patch.

In one variant of the eye patch according to the invention, the sensor unit can be assigned means for an acoustic signal output, whereby the wearer of the eye patch can receive a signal to carry out a change in position in order to restore a more favorable position for the eye.

It can furthermore be expedient for the mentioned microcontroller to include a signal source for generating an absolute or relative time index. Preferably, the data for the time index and/or the movement data and/or position data are storable in the data storage unit and transferable via the data interface.

An external access to the sensor device can optionally be made possible by means of the data interface designed for wireless bidirectional communication or via it, respectively. This data interface designed for wireless bidirectional communication can operate with the so-called Bluetooth standard, for example.

A further variant of the eye patch can provide that the data storage unit that is temporarily connectable with the sensor device takes the form of a microSD card.

At least two of the parts of the sensor unit, for instance, can be integrated into one component in the eye patch according to the invention. The eye patch can optionally be formed by two shells, between which at least one of the parts of the sensor unit is arranged.

According to the invention, the eye patch consists of at least one shell, which largely or completely corresponds to the form of eye patches commonly employed for this purpose. At its outer side, there are at least one position sensor and/or movement sensor, for instance, an inertial sensor, a microcontroller, an energy storage, and a data interface, which are arranged in such a manner as to avoid an impairment of the protective function of the eye patch. The at least one sensor or inertial sensor, respectively, preferably a semiconductor component in MEMS technology, serves for measuring spatial acceleration movements and rotation movements; the data detected in this manner are consequently able to indicate any change in position of the sensor in reference to an existing or specified coordinate system, respectively. In this context it is largely irrelevant in which manner the coordinate system is scaled, i.e. whether the gravity vector is used as determining influence variable, for example; the position of the axes of such a coordinate system is generally freely determinable. The actual position of the eye or of the head, respectively, can now be defined via the detected movement data as deviation from the previously determined movements.

In addition to a processor core, the microcontroller includes at least one working memory and program memory for executing program code and it furthermore provides a data interface or the like with a number of functions for the connection of external elements, such as the inertial sensor. In this manner it is possible to already carry out in the sensor device basic processes of the measurement data acquisition, that is of the detection of the movement data and/or of the position data, by read-out of the values supplied by the inertial sensor, and to pass on and/or to store the data determined in such a manner for later data processing.

The fast progress in technological development in this context, for instance, by increasing the storage capacity and the computing capacity of such a microcontroller, allows the microcontroller to carry out increasingly complex tasks itself, such as data detection, evaluation, storage, and follow-up actions possibly caused thereby, with the microcontroller for this purpose of course having to rely on the communication with an external data processing device, via which, on the one hand, the appropriate program code, etc. reaches the microcontroller via a data interface, and via which, on the other hand, the detected and/or appropriately processed data are passed on by means of the data interface to a data store and/or via a wireless connection to a data processing device.

For the evaluation of the data, an embodiment of the invention can be very helpful in which a time index is generated, for instance by the microcontroller, which time index allows embedding the movement data and/or position data having been provided by the inertial sensor and, according to selected design, processed by the microcontroller, into a temporal context. In this connection, it is largely irrelevant whether the provided time index is absolute, i.e. with real times and dates, such as a present time of the day or the like, or relative, for instance by defining an arbitrary starting time, as long as the assignment can be made of the movement data and/or of the position data to a temporal process, possibly also to external events, such as for instance check-ups.

The data interface can be designed in a plurality of ways; a preferred design is as a radio module, which makes it possible to wirelessly send the data received from the inertial sensor to a data processing device, which provides for the evaluation of the sent data. Ideally, the data interface is formed such that the communication can be carried out bidirectionally so that data can not only be sent, but also received. In this way, for instance, a calibration of the inertial sensor or the microcontroller, respectively, is made possible, as will be explained below in further detail.

The corresponding radio module of the data interface is expediently not designed for communicating over greater distances, but serves primarily for short-distance transmission. Preferred is a module that operates according to the Bluetooth standard, but other existing or future standards are possible as well, as long as they meet the requirements in terms of energy and safety regulations.

In application instances in which the direct and simultaneous use of a data processing device is not possible or not expedient, it is moreover possible to alternatively or additionally equip the data interface with a data storage unit, for instance with a customary microSD card, which is temporarily connected with the data interface for the purpose of data acquisition. This would make it possible to acquire a complete data record, for instance, even if during the recording period there is permanently no connection to a data processing device or if it was interrupted for a certain period of time. For read-in of the data recorded on the data storage unit, the data storage unit can be subsequently removed from the data interface and connected with a data processing device.

As an alternative, it can also be provided to stationarily, i.e. permanently, connect a data storage unit, and thus the sensor device, with the data interface and to provide the data in this manner in the data storage unit of the sensor device in addition to the wireless transfer. As soon as a connection with a data processing device can be made, the read-out of the data from the data storage unit can be carried out, for instance, wirelessly in such an instance.

The electric power required for the operation of the previously mentioned parts of the sensor device has to be supplied by an energy storage and conforms with the number of the elements to be supplied, with their layout, the available space, and expediently also with the weight of the energy storage. Since the predominant number of the parts of the sensor device are based on semiconductor technology and therefore have a rather low weight, the weight of the energy storage is of no little significance, as a high total weight of the eye patch according to the invention not only means an additional burden for the wearer of the eye patch and, according to the embodiment, an additional effort in fastening the eye patch, but it can also affect the healing success if the wearer of the eye patch changes the optimal lying position more than necessary, due to, for instance, the high contact weight.

In this context, the use of a customary button cell has proven advantageous; these are available in different performance levels, inexpensive, and they have a low weight along with a small size.

In a further embodiment of the eye patch it is feasible to additionally arrange an acoustic signal source at the eye patch. Such a signal source can produce, for example, chimes of a simple type, for instance, if the communication connection to the data processing unit is lost, but also for giving out more complex acoustic outputs, such as speech output, provided that the processing capacity of the microcontroller permits this.

In an alternative embodiment of the eye patch according to the invention, it can be provided that two of the elements of the sensor device, for example, inertial sensor and microcontroller, are integrated into one component, or also three or more elements with a corresponding design, such that, for instance, inertial sensor, microcontroller, data interface, and data storage unit, as the case may be, are combined in one component.

A further embodiment of the eye patch can provide that the eye patch consists of two shells, between which at least one of the elements, i.e. one of the parts of the sensor device, is arranged. On the one hand, such an embodiment protects the respective element from damage and on the other hand, it contributes both to increasing the wearing comfort for the wearer of the eye patch by reducing, for instance, the number of parts projecting from the eye patch, and to facilitating the fastening of the eye patch at the eye.

With a higher integration of the elements, it is also possible to arrange a plurality or even all of the elements between the two shells of such an eye patch.

The invention furthermore provides a method for the continuous detection of movement data and/or of position data of a head by means of such an eye patch according to the invention and by means of a data processing device.

The eye patch has a sensor device with at least:
- one sensor for measuring spatial acceleration and rotation movements,
- one microcontroller,
- one energy storage, and
- one data interface designed for wireless bidirectional communication; and the data processing device includes at least:
- one data interface designed for wireless bidirectional communication,
- one computer unit for data processing, and
- one monitor for the visualization of the processed data.

The method provides to fasten an eye patch with the previously described sensor device at the eye, to activate the sensor device, to transfer movement data and/or position data from the sensor device to the data processing device, and to evaluate and visualize the transferred data in the data processing device. In the method, the sensor unit can preferably communicate with a data storage unit, which is permanently and/or temporarily connectable with the sensor unit, in which data storage unit the movement data and/or position data can be stored in addition or as an alternative to the data transmission via the data interface. The sensor unit can moreover include a signal source for generating an absolute or relative time index, which is transmitted to the data processing device and/or stored in the data storage unit together with the movement data and/or with the position data. These data, which are stored in the data storage unit (26,) are transmitted to the data processing device (30) independently of the data interface (22) designed for wireless bidirectional communication. Optionally, the movement data and/or position data can be scaled to at least one freely determinable desired value within an arbitrary coordinate system. It can also be provided that any deviation of the movement data and/or of the position data from the at least one desired value can trigger at least one action. An expedient variant has proven one in which the at least one action involves a visual and/or acoustic information and/or information represented by at least one vibration signal. Other signal variants are, however, also conceivable and can likewise fulfill the desired purpose.

The method provides a calibration of the sensor for the unique determination of the position of the head, with the sensor unit being arranged in a specific orientation. The calibration is carried out after putting on the eye patch and by way of determining the position of the body axis in relation to the sensor device, in which position the position of the head can be uniquely determined.

With this calibration, the sensor position can be adjusted to the intended reference system. Since the anatomy of the bones around the human eye varies, the sensor can assume an individual orientation in relation to the head for each patient after being affixed. Possible problems resulting from these deviations can be solved by registering the sensor orientation immediately after the surgery in a lying position. For operation-related reasons, this is carried out in a flat dorsal position; the red light reflex of the surgical microscope in a vertically standing position can, for instance, serve for verification. For the complete definition of the sensor orientation, the position of the body axis in relation to the sensor is also required; this can be attained by letting the patient sit up (for example, by means of the clinic bed). With this second orientation, the position of the sensor to the head is determined, strictly speaking overdetermined, and a measure for the quality of the calibration can additionally be specified, for the instance that the rotational axis of the sitting position, for example, is not directly located in the horizontal.

For the purpose of instructing/monitoring and recording the therapy-relevant postoperative head posture of a patient, the invention proposes to provide a customary, hard eye patch (which has to be worn in any case) with an inertial sensor as well as with a microprocessor with radio module. A button cell or lithium battery, respectively, or the like, should additionally be arranged for the power supply of these elements. The inertial sensor, consisting of a triaxial accelerometer as well as a triaxial gyroscope, measures the movement and position of the patient. The measured data are processed by the microprocessor and transmitted via radio (for example via Bluetooth) to a data acquisition instrument, for instance to a smartphone, a tablet, or a computer. By means of optical/acoustic or vibration signals, a specifically programmed software can instruct the patient as to the optimal orientation of the head and/or output a warning on the basis of the received data in the instance of a deviation from this position. At the same time, the sensor data can also be recorded and evaluated in the context of clinical trials or for the purpose of quality control of the course of healing. The applicability of the invention primarily lies in the postoperative treatment after certain eye surgeries (for example, pars plana vitrectomy in a retinal detachment and a macular hole or lamellar keratoplasties), when the position of the head is of significance for the course of healing.

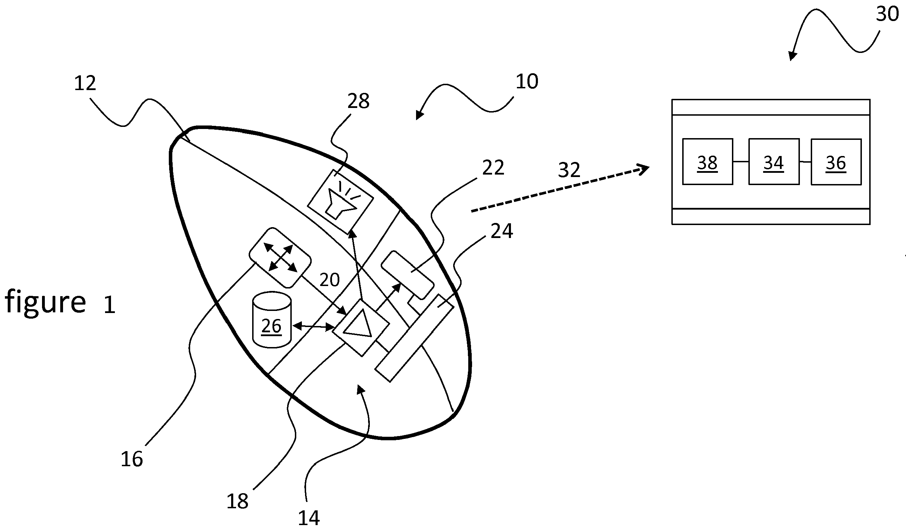

As already mentioned above, the eye patch can have at least one sensor unit or one sensor module, respectively. This phrasing is generally to be understood to the effect that instead of the mentioned sensor unit or the mentioned sensor module, respectively, two or more sensor units or sensor modules, respectively, can also be present, with the microcontroller processing their data. If a sensor unit, a sensor device, or a sensor module is referred to anywhere in the previous description or also in the following description of the figure, which takes reference to one exemplary embodiment, this phrasing therefore also comprises the option of a plurality of sensor units, a plurality of sensor devices, or a plurality of sensor modules, respectively, without this having to be separately mentioned in each case.

In the following passages, the attached figure illustrates in further detail an exemplary embodiment of the invention and its advantages. The size ratios of the individual elements in the figure do not necessarily reflect the real size ratios. It is to be understood that in some instances various aspects of the invention may be shown exaggerated or enlarged in relation to other elements to facilitate an understanding of the invention.

The figure displays a schematic perspective view of an embodiment variant of the eye patch according to the invention with the interacting elements of the sensor unit integrated therein.

It should be understood that the detailed description and specific example of the device according to the invention, while indicating a preferred embodiment, is intended for purposes of illustration only and is not intended to limit the scope of the invention.

The schematic perspective view of the only Figure shows an embodiment variant of an eye patch 10 according to the invention, which serves for covering a human eye (or also an animal eye) and for shielding it from mechanical, climatic influences and/or from electromagnetic radiation, in particular from light radiation. Not least, the eye patch 10 according to the invention due to its provisions, however, also serves to support therapeutic measures, in particular since a desired spatial orientation of the eye can be detected and deviations from a specified orientation can be identified or also prevented, respectively, as the case may be. The eye patch 10 has a shell 12, which partially, in particular completely, covers the eye and its surrounding, which shell 12 is made of bending-resistant material, as well as a sensor unit 14, which comprises at least:
- one position sensor and/or movement sensor or one so-called inertial sensor 16, respectively, for the continuous acquisition of movement data and/or of position data of the eye patch 10;
- one microcontroller 18 for processing the output signals 20 of the at least one position sensor and/or movement sensor or of the inertial sensor 16, respectively;
- one data interface 22 designed for wireless bidirectional communication; as well as
- one electrical energy storage 24 for the electrical energy supply of at least the microcontroller 18 and the data interface 22.

Furthermore, the eye patch 10 comprises a data storage unit 26 assigned to the sensor unit 14, which data storage unit 26 can be permanently and/or temporarily coupled with the position sensor and/or with the movement sensor 16, and which serves for the storage of the movement data and/or of the position data detected by the sensor 16. The sensor unit 14 furthermore includes means 28 for the output at least of an acoustic signal, for example, a loudspeaker or the like. Moreover, the microcontroller 18 can have a signal source for generating an absolute or relative time index. This time index and/or the movement data and/or position data can be stored in the data storage unit 26 and also be optionally transferred via the data interface 22.

An external access to the sensor unit 14 can optionally be made possible via the data interface 22 designed for wireless bidirectional communication. The data interface 22 designed for wireless bidirectional communication can, in particular, operate with the so-called Bluetooth standard.

The data storage unit 26, which is temporarily connectable with the sensor device 14, can be a microSD card, for instance.

At least two of the parts of the sensor unit 14 can preferably be integrated into one component in the eye patch.

In an alternative variant of the eye patch, the eye patch can be formed by two shells, as the case may be, between which at least one of the parts of the sensor unit 14 is arranged.

The present invention provides a method for the continuous detection of movement data and/or of position data of a human head by means of the previously described eye patch 10 and of a data processing device 30 arranged remotely therefrom. The data interface 22 designed for wireless bidirectional communication can communicate wirelessly with the data processing device 30. For this purpose, the data processing device 30 receives wireless data signals 32 from the data interface 22, which is part of the sensor unit 14 of the eye patch 10. The data processing device comprises at least one computer unit 34 for data processing, one monitor 36 for the visualization of the received and processed data 32 and one data interface 38 designed for wireless bidirectional communication, which communicates with the data interface 22 of the eye patch 10. The method according to the invention provides the following steps: first, the eye patch 10 is positioned together with the sensor device 14 at the eye to be monitored, after which the activation of the sensor device 14 can be carried out. The movement data and/or position data detected by the sensor device 14 are transmitted to the data processing device 30; the data processing device 30 evaluates the transferred movement data and/or position data and visualizes the evaluated movement data and/or position data. The data stored in the data storage unit 26 can optionally be transmitted to the data processing device 30 independently of the data interface 22, 38 designed for wireless bidirectional communication, for example, by removing a memory card from the eye patch 10 and positioning it in the data processing device 30.

If required, the movement data and/or position data can be scaled to at least one freely determinable desired value within an arbitrary coordinate system. It can additionally be provided that any deviation of the movement data and/or of the position data from the at least one desired value can trigger at least one action. This action can involve, for instance, a visual and/or acoustic information and/or information represented by at least one vibration signal.

Furthermore, a calibration of the sensor unit 14, which is arranged in a specific orientation, can be carried out after putting on the eye patch 10 and by way of determining the position of the body axis in relation to the sensor device 14, whereby the position of the eye patch in relation to the position of the head can be uniquely determined.

## Claims

1. An eye patch (10) for at least partially covering a human or animal eye and for shielding it from mechanical and/or climatic influences and/or from electromagnetic radiation, in particular from light radiation, wherein the eye patch (10) has at least one shell (12) of bending-resistant material, which shell (12) at least partially covers the eye and its surrounding, as well as a sensor unit (14), which comprises at least:
- one data interface (22) designed for wireless bidirectional communication or data transfer, respectively, as well as
- one electrical energy storage (24) for the electrical energy supply of at least the microcontroller (18) and the data interface (22),
**characterized in that** the at least one sensor unit (14) further comprises at least
- one sensor (16) for the continuous acquisition of movement data of the eye patch (10) by measuring spatial acceleration movements and rotation movements,
- one microcontroller (18) for processing the output signals (20) of the at least one sensor (16).

2. The eye patch as recited in claim 1, in which the sensor unit (14) is assigned a data storage unit (26) that is permanently and/or temporarily couplable with the sensor (16) for the purpose of the storage of at least a part of the movement data acquired by the sensor (16).

3. The eye patch as recited in claim 1 or 2, in which the sensor unit (14) includes means (28) for the output of an acoustic signal.

4. The eye patch as recited in one of the previous claims wherein the microcontroller (22) includes a signal source for generating an absolute or relative time index.

5. The eye patch as recited in claim 4 wherein time index and/or movement data and/or position data are storable in the data storage unit (26) and transferable via the data interface (22).

6. The eye patch as recited in one of the previous claims wherein an external access to the sensor device (16) is carried out or made possible, respectively, via the data interface (22), which is designed for wireless bidirectional communication.

7. The eye patch as recited in one of the previous claims wherein at least two of the parts of the sensor unit (14) are integrated into one component.

8. The eye patch as recited in one of the previous claims wherein the eye patch (10) is formed by two shells (12), between which at least one of the parts of the sensor unit (14) is arranged.

9. A method for the continuous detection of movement data and/or of position data of a human or animal head by means of an eye patch (10) and by means of a data processing device (30), wherein the eye patch (10) has at least one sensor unit (14) with at least:
- one energy storage (24), and
- one data interface (22) designed for wireless bidirectional communication;
and wherein the data processing device (30) comprises at least:
- one computer unit (34) for data processing,
- one monitor (36) for the visualization of the processed data (32),
- and one data interface (38) designed for wireless bidirectional communication,
**characterized in that** the at least one sensor unit (14) further comprises at least
- one sensor (16) for measuring spatial acceleration movements and rotation movements,
- one microcontroller (18);
and **in that** the method has at least the following steps:
- positioning the eye patch (10) and the sensor device (14) at the eye,
- activating the sensor device (14),
- transferring the movement data detected by the sensor device (14) to the data processing device (30),
- evaluating the transferred movement data in the data processing device (30), and
- visualizing and/or storing the evaluated movement data and/or position data.

10. The method as recited in claim 9 wherein the sensor unit (14) communicates with a data storage unit (26), which is permanently and/or temporarily connectable with the sensor unit (14), in which data storage unit (26) the movement data and/or position data can be stored in addition or as an alternative to the data transmission via the data interface (22).

11. The method as recited in claim 10 wherein the sensor unit (14) includes a signal source for generating an absolute or relative time index, which is transmitted to the data processing device (30) and/or stored in the data storage unit (26) together with the movement data and/or with the position data.

12. The method as recited in claim 10 or 11 wherein the data stored in the data storage unit (26) are transmitted to the data processing device (30) independently of the data interface (22) designed for wireless bidirectional communication.

13. The method as recited in one of the claims 9 to 12 wherein the movement data and/or position data can be scaled to at least one freely determinable desired value within an arbitrary coordinate system.

14. The method as recited in claim 13 wherein each deviation of the movement data and/or of the position data from the at least one desired value can trigger at least one action, and wherein this at least one action involves, in particular, visual and/or acoustic information and/or information represented by at least one vibration signal.

15. The method as recited in one of the claims 9 to 14, in which a calibration of the sensor device for the unique determination of the position of the head is carried out after putting on the eye patch (10) by determining the position of the body axis in relation to the sensor device (14).

## Patentansprüche

1. Augenklappe (10) zur zumindest teilweisen Abdeckung eines menschlichen oder tierischen Auges und zu dessen Abschirmung vor mechanischen und/oder klimatischen Einflüssen und/oder vor elektromagnetischer Strahlung, insbesondere Lichtstrahlung, wobei die Augenklappe (10) wenigstens eine das Auge und dessen Umgebung zumindest partiell abdeckende Schale (12) aus biegesteifem Material sowie eine Sensoreinheit (14) aufweist, welche zumindest umfasst:
- eine zur drahtlosen, bidirektionalen Kommunikation bzw. Datenübertragung ausgebildete Datenschnittstelle (22), sowie
- einen elektrischen Energiespeicher (24) zur elektrischen Energieversorgung zumindest des Mikrocontrollers (18) und der Datenschnittstelle (22),
**dadurch gekennzeichnet, dass** die wenigstens eine Sensoreinheit (14) zumindest weiterhin umfasst
- einen Sensor (16) zur kontinuierlichen Erfassung von Bewegungsdaten der Augenklappe (10) durch Messung von Beschleunigungs- und Drehbewegungen im Raum,
- einen Mikrocontroller (18) zur Verarbeitung der Ausgangssignale (20) des wenigstens einen Sensors (16).

2. Augenklappe nach Anspruch 1, bei welcher der Sensoreinheit (14) eine dauerhafte und/oder temporär mit dem Sensor (16) koppelbare Datenspeichereinheit (26) zur Speicherung zumindest eines Teils der vom Sensor (16) erfassten Bewegungsdaten zugeordnet ist.

3. Augenklappe nach Anspruch 1 oder 2, bei der die Sensoreinheit (14) Mittel (28) zur Ausgabe eines akustischen Signals enthält.

4. Augenklappe nach einem der vorstehenden Ansprüche, wobei der Mikrocontroller (22) eine Signalquelle zur Erzeugung eines absoluten oder relativen Zeitindex enthält.

5. Augenklappe nach Anspruch 4, wobei Zeitindex und/oder Bewegungs- und/oder Lagedaten in der Datenspeichereinheit (26) speicherbar und über die Datenschnittstelle (22) übertragbar sind.

6. Augenklappe nach einem der vorstehenden Ansprüche, wobei über die zur drahtlosen, bidirektionalen Kommunikation ausgebildete Datenschnittstelle (22) ein externer Zugriff auf die Sensoreinrichtung (14) erfolgt bzw. ermöglicht ist.

7. Augenklappe nach einem der vorstehenden Ansprüche, wobei wenigstens zwei der Bestandteile der Sensoreinheit (14) in einem Bauteil integriert sind.

8. Augenklappe nach einem der vorstehenden Ansprüche, wobei die Augenklappe (10) durch zwei Schalen (12) gebildet ist, zwischen denen wenigstens einer der Bestandteile der Sensoreinheit (14) angeordnet ist.

9. Verfahren zur kontinuierlichen Ermittlung von Bewegungs- und/oder Lagedaten eines menschlichen oder tierischen Kopfes mittels einer Augenklappe (10) und einer Datenverarbeitungseinrichtung (30), wobei die Augenklappe (10) wenigstens eine Sensoreinheit (14) aufweist mit wenigstens
- einem Energiespeicher (24) und
- einer zur drahtlosen, bidirektionalen Kommunikation ausgebildete Datenschnittstelle (22),
und wobei die Datenverarbeitungseinrichtung (30) wenigstens umfasst:
- eine Rechnereinheit (34) zur Datenverarbeitung,
- einen Bildschirm (36) zur Visualisierung der verarbeiteten Daten (32)
- und eine zur drahtlosen, bidirektionalen Kommunikation ausgebildete Datenschnittstelle (38),
**dadurch gekennzeichnet, dass** die wenigstens eine Sensoreinheit (14) weiterhin wenigstens umfasst:
- einen Sensor (16) zur Messung von Beschleunigungs- und Drehbewegungen im Raum,
- einen Mikrocontroller (18),
und dass das Verfahren wenigstens die folgenden Schritte aufweist:
- Positionieren der Augenklappe (10) und der Sensoreinrichtung (14) am Auge,
- Aktivieren der Sensoreinrichtung (14),
- Übertragung der von der Sensoreinrichtung (14) ermittelten Bewegungsdaten an die Datenverarbeitungseinrichtung (30),
- Auswertung der übertragenen Bewegungsdaten in der Datenverarbeitungseinrichtung (30), und
- Visualisierung und/oder Speicherung der ausgewerteten Bewegungs-und/oder Lagedaten.

10. Verfahren nach Anspruch 9, wobei die Sensoreinheit (14) mit einer dauerhaft und/oder temporär mit der Sensoreinheit (14) verbindbaren Datenspeichereinheit (26) kommuniziert, in welcher die Bewegungs- und/oder Lagedaten zusätzlich oder alternativ zur Datenübermittlung über die Datenschnittstelle (22) gespeichert werden können.

11. Verfahren nach Anspruch 10, wobei die Sensoreinheit (14) eine Signalquelle zur Erzeugung eines absoluten oder relativen Zeitindex enthält, welcher zusammen mit den Bewegungs- und/oder Lagedaten an die Datenverarbeitungseinrichtung (30) übermittelt und/oder in der Datenspeichereinheit (26) gespeichert wird.

12. Verfahren nach Anspruch 10 oder 11, wobei die in der Datenspeichereinheit (26) gespeicherten Daten unabhängig von der zur drahtlosen, bidirektionalen Kommunikation ausgebildeten Datenschnittstelle (22) an die Datenverarbeitungseinrichtung (30) übermittelt werden.

13. Verfahren nach einem der Ansprüche 9 bis 12, wobei die Bewegungs- und/oder Lagedaten innerhalb eines beliebigen Koordinatensystems auf wenigstens einen frei bestimmbaren Sollwert normiert werden können.

14. Verfahren nach Anspruch 13, wobei jede Abweichung der Bewegungs- und/oder Lagedaten vom wenigstens einen Sollwert wenigstens eine Aktion auslösen kann, und wobei es sich bei der wenigstens einen Aktion insbesondere um einen visuellen und/oder akustischen und/oder durch wenigstens ein Vibrationssignal dargestellten Hinweis handelt.

15. Verfahren nach einem der Ansprüche 9 bis 14, bei dem eine Kalibrierung der Sensoreinrichtung nach Anbringen der Augenklappe (10) durch Bestimmung der Lage der Körperachse in Relation zur Sensoreinrichtung (14) zur eindeutigen Bestimmung der Lage des Kopfes erfolgt.

## Revendications

1. Cache-œil (10) destiné à recouvrir au moins en partie un œil humain ou animal et à protéger celui-ci d'influences mécaniques et/ou climatiques et/ou de rayonnement électromagnétique, en particulier de rayonnement lumineux, dans lequel ledit cache-œil (10) présente au moins une coque (12) en matériau résistant à la flexion, laquelle coque (12) recouvre au moins en partie l'œil et son environnement, ainsi qu'une unité de capteur (14) qui comprend au moins:
- une interface de données (22) conçue pour la communication bidirectionnelle sans fil ou bien pour la transmission de données, ainsi qu'
- un accumulateur d'énergie électrique (24) pour alimenter en énergie électrique au moins le microcontrôleur (18) et l'interface de données (22),
**caractérisé par le fait que** ladite au moins une unité de capteur (14) comprend en outre au moins
- un capteur (16) destiné à saisir en continu des données de mouvement du cache-œil (10) en mesurant des mouvements d'accélération et des mouvements de rotation dans l'espace,
- un microcontrôleur (18) destiné à traiter les signaux de sortie (20) dudit au moins un capteur (16).

2. Cache-œil selon la revendication 1, dans lequel à ladite unité de capteur (14) est associée une unité de stockage de données (26) qui peut être couplée en permanence et/ou temporairement au capteur (16) afin de stocker au moins une partie des données de mouvement saisies par le capteur (16).

3. Cache-œil selon la revendication 1 ou 2, dans lequel ladite unité de capteur (14) comprend des moyens (28) destinés à délivrer un signal acoustique.

4. Cache-œil selon l'une quelconque des revendications précédentes, dans lequel le microcontrôleur (22) comprend une source de signal pour générer un indice temporel absolu ou relatif.

5. Cache-œil selon la revendication 4, dans lequel l'indice temporel et/ou les données de mouvement et/ou de position peuvent être stockés dans l'unité de stockage de données (26) et être transmis via l'interface de données (22).

6. Cache-œil selon l'une quelconque des revendications précédentes, dans lequel un accès externe au dispositif de capteur (14) se fait ou bien est rendu possible via l'interface de données (22) qui est conçue pour la communication bidirectionnelle sans fil.

7. Cache-œil selon l'une quelconque des revendications précédentes, dans lequel au moins deux des parties de l'unité de capteur (14) sont intégrées à un composant.

8. Cache-œil selon l'une quelconque des revendications précédentes, dans lequel le cache-œil (10) est constitué par deux coques (12) entre lesquelles est disposée au moins l'une des parties de l'unité de capteur (14).

9. Procédé destiné à saisir en continu des données de mouvement et/ou des données de position d'une tête humaine ou animale au moyen d'un cache-œil (10) et au moyen d'un dispositif de traitement de données (30), dans lequel ledit cache-œil (10) présente au moins une unité de capteur (14) ayant au moins:
- un accumulateur d'énergie (24), et
- une interface de données (22) conçue pour la communication bidirectionnelle sans fil;
et dans lequel le dispositif de traitement de données (30) comprend au moins:
- une unité informatique (34) pour le traitement de données,
- un écran (36) pour la visualisation des données traitées (32),
- et une interface de données (38) conçue pour la communication bidirectionnelle sans fil,
**caractérisé par le fait que** ladite au moins une unité de capteur (14) comprend en outre au moins:
- un capteur (16) destiné à mesurer des mouvements d'accélération et des mouvements de rotation dans l'espace,
- un microcontrôleur (18);
et que le procédé comprend au moins les étapes suivantes consistant à:
- positionner le cache-œil (10) et le dispositif de capteur (14) au niveau de l'œil,
- activer le dispositif de capteur (14),
- transmettre les données de mouvement saisies par le dispositif de capteur (14) au dispositif de traitement de données (30),
- évaluer les données de mouvement transmises dans le dispositif de traitement de données (30), et
- visualiser et/ou stocker les données de mouvement et/ou les données de position évaluées.

10. Procédé selon la revendication 9, dans lequel l'unité de capteur (14) communique avec une unité de stockage de données (26) qui peut être connectée en permanence et/ou temporairement à l'unité de capteur (14), dans laquelle unité de stockage de données (26) les données de mouvement et/ou les données de position peuvent être stockées en plus de ou en alternative à la transmission de données via l'interface de données (22).

11. Procédé selon la revendication 10, dans lequel l'unité de capteur (14) comprend une source de signal pour générer un indice temporel absolu ou relatif qui est transmis au dispositif de traitement de données (30) et/ou stocké dans l'unité de stockage de données (26) conjointement avec les données de mouvement et/ou avec les données de position.

12. Procédé selon la revendication 10 ou 11, dans lequel les données stockées dans l'unité de stockage de données (26) sont transmises au dispositif de traitement de données (30) indépendamment de l'interface de données (22) conçue pour la communication bidirectionnelle sans fil.

13. Procédé selon l'une quelconque des revendications 9 à 12, dans lequel les données de mouvement et/ou les données de position peuvent être normalisées à au moins une valeur de consigne librement déterminable, dans un système de coordonnées quelconque.

14. Procédé selon la revendication 13, dans lequel chaque écart des données de mouvement et/ou des données de position par rapport à ladite au moins une valeur de consigne peut déclencher au moins une action, et dans lequel cette au moins une action implique, en particulier, un renseignement visuel et/ou acoustique et/ou un renseignement représenté par au moins un signal de vibration.

15. Procédé selon l'une quelconque des revendications 9 à 14, dans lequel un calibrage du dispositif de capteur pour la détermination unique de la position de la tête est effectué après avoir mis le cache-œil (10) en déterminant la position de l'axe du corps en relation au dispositif de capteur (14).
